# EUROPÄISCHE PATENTANMELDUNG

(11) **EP 1 400 506 A2**
(43) Veröffentlichungstag der Anmeldung: **24.03.2004**
(21) Anmeldenummer: 03019635.6
(22) Anmeldetag: 05.09.2003
(51) Int. Cl.: C07C 67/24, C07C 69/007

(54) **Verfahren zur Herstellung von Carbonsäurebenzylestern**

(30) Priorität: 18.09.2002 DE 10243200
(71) Anmelder: Bayer Chemicals AG, 51368 Leverkusen (DE)
(72) Erfinder: Ooms, Pieter, Dr., 47800 Krefeld (DE); Schenke, Bernd-Ulrich, 46236 Bottrop (DE)

(57) **Zusammenfassung**

Carbonsäurebenzylester können durch Umsetzung von Dibenzylether mit Carbonsäureanhydriden in Gegenwart einer oder mehrerer, bevorzugt einer Säure, gegebenenfalls aufgebracht auf einem oder mehreren, bevorzugt einem, Träger, als Katalysator hergestellt werden.

## Beschreibung

Die vorliegende Erfindung betrifft ein Verfahren zur Herstellung von Carbonsäurebenzylestem durch Reaktion von Dibenzylether mit Carbonsäureanhydriden in Gegenwart einer oder mehrerer, bevorzugt einer, Säure als Katalysator.

Benzylacetat, die Hauptkomponente des Jasminöls, ist ein wichtiger Riechstoff zur Herstellung von Duftkompositionen und Ausgangsprodukt für die Herstellung von Fruchtethem.

Die Herstellung von Benzylacetat durch Veresterung von Benzylalkohol mit Essigsäure ist seit langem bekannt.

Benzylacetat kann auch durch Umsetzung von Benzylchlorid mit Alkaliacetaten gegebenenfalls in Gegenwart von Phasentransferreagenzien hergestellt werden (Wang et al., Chem. Eng. Commun., 100, S.135 bis 147 (1991)). Nachteilig ist die Bildung von Salzen, die entsorgt werden müssen und somit die Wirtschaftlichkeit dieser Verfahren verringern.

DD-A5-286 577 beschreibt die Herstellung von Benzylacetat durch Umsetzung von Dibenzylether mit Essigsäureanhydrid. Nachteilig sind die drastischen Reaktionsbedingungen (300°C/20 Mpa) und die nur mäßigen Ausbeuten.

Es bestand also die Aufgabe, ausgehend von Dibenzylether ein Verfahren zur Herstellung von Carbonsäurebenzylestern zu entwickeln, welches unter milden Reaktionsbedingungen durchführbar ist und kostengünstig zu guten Ausbeuten führt.

Es wurde ein Verfahren zur Herstellung von Carbonsäurebenzylestern der Formel 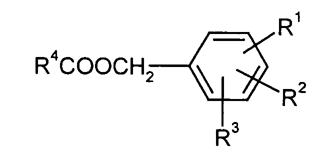 worin
- R¹ bis: R³ gleich oder verschieden sind und für Wasserstoff, C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
- R⁴: für Wasserstoff, C₁-C₂₀-Alkyl, C₂-C₂₀-Alkenyl, C₇-C₁₄-Aralkyl, C₆-C₁₂-Aryl, C₁-C₆-Halogenalkyl, C₂-C₆-Halogenalkenyl- oder C₆-C₁₂-Halogenaryl,
stehen,
aus Dibenzylethern gefunden, das dadurch gekennzeichnet ist, dass Dibenzylether der Formel 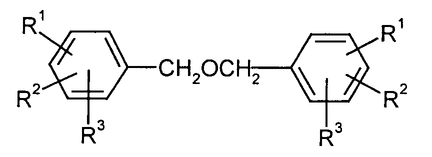 worin
R¹, R² und R³ die obengenannte Bedeutung haben,
oder Gemische aus Dibenzylethem und Benzylalkoholen der Formel 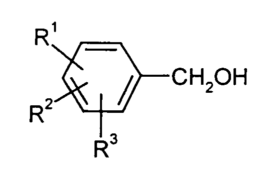 worin
R¹, R² und R³ die obengenannte Bedeutung haben
mit Carbonsäureanhydriden der Formel

(R⁴CO)₂O

worin
R⁴ die obengenannte Bedeutung hat,
in Gegenwart mindestens einer Säure als Katalysator, der gegebenenfalls auf einem Träger aufgebracht sein kann, umgesetzt werden.

Als Katalysator können eine oder mehrere Säuren verwendet werden. Vorzugsweise wird eine Säure verwendet. Der Katalysator kann dabei auf einem oder mehreren Trägern aufgebracht sein. Vorzugsweise wird ein Träger verwendet.

Das erfindungsgemäße Verfahren lässt sich kostengünstig und unter milden Reaktionsbedingungen durchführen.

Die Reste R¹ bis R³ haben im allgemeinen die folgende Bedeutung:

Alkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen. Beispielsweise seien Methyl, Ethyl, Propyl, iso-Propyl, Butyl, iso-Butyl, Pentyl, iso-Pentyl, Hexyl und iso-Hexyl genannt. Bevorzugt sind Methyl, Ethyl, Propyl, iso-Propyl, Butyl, Pentyl und Hexyl, im besonderen bevorzugt sind Methyl und Ethyl.

Alkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen. Beispielsweise seien Methoxy, Ethoxy, Propoxy, iso-Propoxy, Butoxy, iso-Butoxy, Pentoxy, iso-Pentoxy, Hexoxy und iso-Hexoxy genannt. Bevorzugt sind Methoxy, Ethoxy, Propoxy, iso-Propoxy, Butoxy, iso-Butoxy, Pentoxy und Hexoxy, im besonderen bevorzugt sind Methoxy und Ethoxy.

Halogenalkyl bedeutet im allgemeinen ein geradkettiger oder verzweigter Kohlenwasserstoffrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethyl, Fluormethyl, Difluormethyl, Trifluormethyl, Fluorethyl, Fluorpropyl und Hexafluorbutyl genannt. Bevorzugt sind Fluormethyl, Difluormethyl, Trifluormethyl, Fluorethyl, Fluorpropyl und Hexafluorbutyl, im besonderen bevorzugt sind Fluormethyl und Trifluormethyl.

Halogenalkoxy bedeutet im allgemeinen ein geradkettiger oder verzweigter Alkoxyrest mit 1 bis 6, bevorzugt 1 bis 4, im besondere bevorzugt 1 oder 2 Kohlenstoffatomen mit 1 bis 10, bevorzugt 1 bis 8, im besondere bevorzugt mit 1 bis 5 Halogenatomen. Beispielsweise seien Chlormethoxy, Fluormethoxy, Difluormethoxy, Trifluormethoxy, Fluorethoxy, Fluorpropoxy und Hexafluorbutoxy genannt. Bevorzugt sind Chlormethoxy, Fluormethoxy, Trifluormethoxy, Fluorethoxy, Fluorpropoxy und Hexafluorbutoxy, im besonderen bevorzugt sind Fluormethoxy und Trifluormethoxy.

Halogen bedeutet im allgemeinen Fluor, Chlor, Brom und Jod, bevorzugt Fluor, Chlor und Brom, im besonderen Fluor und Chlor.

Ganz besonders bevorzugte Substituenten für R¹ bis R³ sind Wasserstoff, Methyl, Trifluormethyl, Methoxy, Fluor oder Chlor.

Nach dem erfindungsgemäßen Verfahren können beispielsweise die folgenden Carbonsäurebenzylester hergestellt werden:

Ameisensäurebenzylester, Essigsäurebenzylester, Chloressigsäurebenzylester, Propionsäurebenzylester, Buttersäurebenzylester, Pentansäurebenzylester, Hexansäurebenzylester, Heptansäurebenzylester, Octansäurebenzylester, Nonansäurebenzylester, Decansäurebenzylester, Undecansäurebenzylester, Dodecansäurebenzylester, Tridecansäurebenzylester, Tetradecansäurebenzylester, Pentadecansäurebenzylester, Hexadecansäurebenzylester, Heptadecansäurebenzylester, Oktadecansäurebenzylester, Nonadecansäurebenzylester, Phenylessigsäure-benzylester, Zimtsäurebenzylester, Benzoesäurebenzylester, 3-Chlorbenzoesäure-benzylester, 2-Hydroxybenzoesäurebenzylester, 3-Hydroxybenzoesäurebenzylester, 4-Hydroxybenzoesäurebenzylester, 3-Chlor-2-hydroxybenzoesäurebenzylester, 4-Chlor-2-hydroxybenzoesäurebenzylester, 5-Chlor-2-hydroxybenzoesäurebenzylester, 6-Chlor-2-hydroxybenzoesäurebenzylester, 3-Brom-2-hydroxybenzoesäurebenzylester, 3-Fluor-2-hydroxybenzoesäurebenzylester, 4-Fluor-2-hydroxybenzoesäurebenzylester, 2-Fluor-3-hydroxybenzoesäurebenzylester, 2-Fluor-4-hydroxybenzoesäurebenzylester, 3-Fluor-2-hydroxybenzoesäurebenzylester, 2-Fluor-5-hydroxybenzoesäurebenzylester, 2-Fluor-6-hydroxybenzoesäurebenzylester, 2-Hydroxy-3-methylbenzoesäurebenzylester, 2-Hydroxy-4-methylbenzoesäurebenzylester, 3-Hydroxy-2-methylbenzoesäurebenzylester, 4-Hydroxy-2-methylbenzoesäurebenzylester, 2-Fluor-6-hydroxy-4-methoxybenzoesäurebenzylester, 3-Trifluormethyl-2-hydroxybenzoesäurebenzylester, 4-Trifluormethyl-2-hydroxybenzoesäurebenzylester, 2-Trifluormethyl-3-hydroxybenzoesäurebenzylester, 2-Fluorethyl-4-hydroxybenzoesäurebenzylester und 4-Fluorbutyl-2-hydroxybenzoesäurebenzylester.

Bei dem im erfindungsgemäßen Verfahren eingesetzten Dibenzylether werden unsubstituierten oder substituierten Dibenzylether eingesetzt.

Besonders bevorzugt wird unsubstituierter Dibenzylether eingesetzt.

In einer bevorzugten Ausführungsform der Erfindung handelt es sich bei dem Dibenzylether um einen substituierten Dibenzylether, welcher einen oder mehrere Substituenten aus der Reihe C₁-C₆-Alkyl, C₁-C₆-Alkoxy-, CN, CO(C₁-C₆-Alkyl), NO₂ oder Halogen trägt.

In das erfindungsgemäßen Verfahren können Dibenzylether oder Dibenzylether/Benzylalkoholgemische, wie sie beispielsweise bei der Herstellung von Benzylalkohol aus Benzylchlorid anfallen, eingesetzt werden. Der Gehalt an Dibenzylether kann 50 bis 100 %, bevorzugt 60 bis 99 %, besonders bevorzugt 70 bis 98 % sein.

Bei den im erfindungsgemäßen Verfahren eingesetzten Carbonsäureanhydriden handelt es sich um geradkettige und verzweigte, gesättigte und ungesättigte Alkyl-, Aralkyl- und Arylcarbonsäureanhydride mit 1 bis 20 C-Atomen, wie beispielsweise Essigsäureanhydrid, Propionsäureanhydrid, Isobuttersäureanhydrid, Valeriansäureanhydrid, Isovaleriansäureanhydrid, Capronsäureanhydrid, Heptansäureanhydrid, Caprylsäureanhydrid, Nonansäureanhydrid, Caprinsäureanhydrid, Undecansäureanhydrid, Laurinsäureanhydrid, Tridecansäureanhydrid, Myristinsäureanhydrid, Palmitinsäureanhydrid, Stearinsäureanhydrid, Ölsäureanhydrid, Linolsäureanhydrid, Chloressigsäureanhydrid, Linolensäureanhydrid, Acrylsäureanhydrid, Methacrylsäureanhydrid, Zimtsäureanhydrid, Phenylessigsäureanhydrid, Benzoesäureanhydrid oder Salicylsäureanhydrid als auch gemischte Anhydride wie beispielsweise das gemischte Anhydrid von Ameisensäure und Essigsäure. Bevorzugt sind Carbonsäureanhydride mit 1 bis 15 C-Atomen, besonders bevorzugt 1 bis 10 C-Atomen. Ganz besonders bevorzugte Carbonsäureanhydride sind Essigsäureanhydrid, Chloressigsäureanhydrid, Propionsäureanhydrid, Buttersäureanhydrid, Isobuttersäureanhydrid, Valeriansäureanhydrid, Hexansäureanhydrid und Benzoesäureanhydrid.

Im erfindungsgemäßen Verfahren werden 1 bis 50 Äquivalente, vorzugsweise 1 bis 25 Äquivalente, besonders bevorzugt 1 bis 15 Äquivalente und ganz besonders bevorzugt 1 bis 10 Äquivalente, Carbonsäureanhydrid, bezogen auf Dibenzylether, eingesetzt.

Im erfindungsgemäßen Verfahren können homogene als auch heterogene Katalysatoren eingesetzt werden.

Geeignete Katalysatoren für das erfindungsgemäße Verfahren sind anorganische Säuren wie beispielsweise Schwefeltrioxid, Schwefelsäure, Chlorwasserstoff, Bromwasserstoff, Jodwasserstoff, Flusssäure, Perchlorsäure, Chlorsulfonsäure oder Phosphorsäure, organische Säuren wie beispielsweise Trifluoressigsäure, Methansulfonsäure, Ethansulfonsäure, Benzolsulfonsäure, 4-Toluolsulfonsäure, Chlorsulfonsäure oder Trifluormethansulfonsäure und Lewissäuren wie beispielsweise Bortrifluorid, Aluminiumchlorid, Aluminiumbromid, Aluminiumiodid, Zinkchlorid, Zinnchlorid, Titanchlorid oder Zirkonchlorid, gegebenenfalls aufgebracht auf einem oder mehreren, bevorzugt einem, Träger.

Bevorzugt sind Schwefeltrioxid, Schwefelsäure, Trifluormethansulfonsäure, 4-Toluolsulfonsäure, Chlorsulfonsäure und Bortrifluorid, besonders bevorzugt Schwefeltrioxid, Schwefelsäure, Trifluormethansulfonsäure, Chlorsulfonsäure und Bortrifluorid, gegebenenfalls aufgebracht auf einem Träger.

Weitere geeignete Katalysatoren für das erfindungsgemäße Verfahren sind Heteropolysäuren der Formel (I)

AₐX_{b}M_{c}O_{d} (I)

worin
- A: für Protonen und/oder für Metallkationen steht
- X: für P, Si, B, Ge, As, I, Se oder Te steht
- M: für W, Mo, V oder Cr steht
- a: für 3, 4, 5 oder 6 steht, so dass die Elektroneutralität der Heteropolysäuren oder deren Salze gegeben ist
- b: für 1 oder 2 steht
- c: für 12 oder 18 steht und
- d: für 40 oder 62 steht
gegebenenfalls aufgebracht auf einem oder mehreren, bevorzugt einem, Träger.

Als geeignete Kationen A sind beispielsweise Kationen der Alkalimetalle wie Lithium, Natrium, Kalium, Rubidium oder Caesium, oder Kationen der Metalle Mangan, Nickel, Cobalt, Kupfer oder Lanthan oder Protonen zu nennen.

Bevorzugte Heteropolysäuren sind Molybdatophosphorsäure, Wolframatophosphorsäure, Vanadatophosphorsäure, Molybdatokieselsäure, Wolframatokieselsäure, Vanadatokieselsäure, besonders bevorzugte Heteropolysäuren sind Molybdatophosphorsäure, Wolframatophosphorsäure, Molybdatokieselsäure und Wolframatokieselsäure, gegebenenfalls aufgebracht auf einem Träger.

Bevorzugte Heteropolysäuren sind weiterhin solche des Keggin-Typs, d.h. Verbindungen der Formel (I), worin b für 1, c für 12 und d für 40 steht und solche des Dawson-Typs, d.h. Verbindungen der Formel (I), worin b für 2, c für 18 und d für 62 steht. Besonders bevorzugte Verbindungen sind A₃[PMo₁₂O₄₀],A₃[PW₁₂O₄₀], A₃[SiMo₁₂O₄₀] und A₃[SiW₁₂O₄₀].

Methoden zur Herstellung der Heteropolysäuren sind bekannt und beispielsweise in Römpp, Lexikon der Chemie Band 3, 10. Auflage, Stuttgart/New York 1997, S. 1741; Chemical Reviews 98, 1998, 1ff oder Catal. Rev. Sci. Eng. 37, 1995, 311ff beschrieben.

In einer bevorzugten Ausführungsform der Erfindung werden als Säuren anorganische Säuren, organische Säuren oder Lewissäuren mit einem pH-Wert von 1 bis 6 verwendet.

Geeignete Träger für das erfindungsgemäße Verfahren sind Oxide oder Sulfate von Elementen der Gruppen II A (Gruppe 2 nach IUPAC), beispielweise Magnesium, Calcium oder Barium, III B (Gruppe 3 nach IUPAC), beispielweise Scandium, Yttrium oder Lanthan, IV B (Gruppe 4 nach IUPAC), beispielsweise Titan, Zirkon oder Hafnium, V B (Gruppe 5 nach IUPAC), beispielsweise Niob oder Tantal, VII B (Gruppe 7 nach IUPAC), beispielsweise Mangan, VIII (Gruppe 8, 9 und 10 nach IUPAC), beispielsweise Eisen oder Nickel, III A (Gruppe 13 nach IUPAC), beispielsweise Al und IV A (Gruppe 14 nach IUPAC), beispielsweise Silizium, Germanium, Zinn oder Blei und Kohlenstoff.

Beispielsweise sind CaO, MgO, ZrO₂, TiO₂, HfO₂, SnO₂, Al₂O₃, SiO₂, Al₂O₃.SiO₂ (Alumosilikate wie Zeolithe oder Schichtsilikate), Nb₂O₅, Ta₂O₅, Fe₂O₃, LaSO₄ oder CaSO₄ und Aktivkohlen zu nennen.

Bevorzugt sind CaO, MgO, ZrO₂, TiO₂, HfO₂, SnO₂, Al₂O₃.SiO₂, Al₂O₃, SiO₂, Nb₂O₅, Ta₂O₅, Fe₂O₃, LaSO₄ oder CaSO₄, im besonderen bevorzugt sind CaO, MgO, SnO₂, ZrO₂, TiO₂, HfO₂, Al₂O₃, SiO₂, Al₂O₃.SiO₂, Nb₂O₅ und Ta₂O₅.

Als Katalysatoren können bevorzugt Schwefeltrioxid, Schwefelsäure und Trifluormethansulfonsäure auf Aktivkohle, CaO, MgO, ZrO₂, TiO₂, HfO₂, SnO₂, Al₂O₃, SiO₂, Al₂O₃.SiO₂ (Alumosilikate wie Zeolithe oder Schichtsilikate), Nb₂O₅, Ta₂O₅, Fe₂O₃, LaSO₄ oder CaSO₄ eingesetzt werden.

Als Katalysatoren können bevorzugt Molybdatophosphorsäure, Wolframatophosphorsäure, Vanadatophosphorsäure, Molybdatokieselsäure, Wolframatokieselsäure oder Vanadatokieselsäure auf Aktivkohle, CaO, MgO, ZrO₂, TiO₂, HfO₂, SnO₂, Al₂O₃, SiO₂, Al₂O₃.SiO₂ (Alumosilikate wie Zeolithe oder Schichtsilikate), Nb₂O₅, Ta₂O₅, Fe₂O₃, LaSO₄ oder CaSO₄ eingesetzt werden.

Ganz besonders als Katalysatoren bevorzugt sind sulfatierte Oxide (Supersäure) wie SO₃ auf CaO, MgO, ZrO₂, TiO₂, HfO₂, SnO₂, Al₂O₃, SiO₂, Al₂O₃.SiO₂, Nb₂O₅, Ta₂O₅ oder Fe₂O₃.

Methoden zur Herstellung sind gut bekannt und beispielsweise beschrieben in Applied Catalysis A, 146, 1996, S. 3 bis 32, Catalysis Today 20, S. 219 bis 256 (1994) und WO 00/64849).

Die Säuren oder deren Hydrate, können auf einen Träger aufgebracht, gegebenenfalls kalziniert, als heterogener Katalysator eingesetzt werden.

Geeignete heterogene Katalysatoren für das erfindungsgemäße Verfahren sind vorzugsweise saure Ionenaustauscher wie beispielsweise Sulfonsäure-Gruppen tragende Polymere, wobei es sich bei den Polymeren beispielsweise um Polystyrole, Styrol-Divinylbenzol-Copolymerisate oder Phenol-Formaldehydharze handeln kann. Bevorzugte saure Ionenaustauscher sind sulfonylierte Polystyrole, sulfonylierte Styrol-Divinylbenzol-Copolymerisate oder sulfonylierte Phenol-Formaldehydharze, ganz besonders bevorzugt sind sulfonylierte Polystyrole.

Weiterhin werden besonders bevorzugt fluorierte oder perfluorierte Sulfonsäure-Gruppen tragende Polymere eingesetzt wie beispielsweise fluorierte oder perfluorierte sulfonylierte Polystyrole, fluorierte oder perfluorierte sulfonylierte Styrol-Divinylbenzol-Copolymerisate oder fluorierte oder perfluorierte sulfonylierte Phenol-Formaldehydharze. Ganz besonders bevorzugt werden fluorierte oder perfluorierte sulfonylierte Polystyrole eingesetzt.

Die Sulfonsäure-Gruppen tragenden Ionenaustauscher sind durch Umsetzung von Polymeren mit Sulfonierungsmitteln wie Schwefelsäure oder Chlorsulfonsäure herstellbar. Die Herstellung ist beispielsweise in Encyclopedia of Polymer Science and Technology Vol. 7, Ed. N.M. Bikales, Interscience Publishers New York, 1967, S. 695 ff beschrieben.

Es können auch Mischungen von sauren Ionenaustauschern eingesetzt werden.

Da aus Mastagli et al., C. r. 232, **1951**, 1848-1849 bekannt ist, dass Dibenzylether in Gegenwart von sulfonierten Phenol-Formaldehydharzen zu Toluol und Benzaldehyd umgesetzt wird, ist die Eignung von sauren Ionenaustauschern als Katalysatoren im erfindungsgemäßen Verfahren besonders überraschend.

Die sauren Ionenaustauscher können in Kugelform vorliegen und Korngrößen von 0,3 bis 3,0 mm Durchmesser aufweisen. Sie können vom Geltyp oder makroporös sein. Ihre Totalkapazität an Säurefunktionen in wasserfeuchter Form mit einem Wassergehalt von ca. 75 bis 85 Gew.-% liegt vorzugsweise bei 0,7 bis 2,1 oder 3,5 bis 5 mval/ml Ionenaustauscher, bezogen auf 1 g Trockensubstanz an Ionenaustauscher.

Geeignete saure Ionenaustauscher sind beispielsweise die unter den folgenden registrierten Handelsnamen vertriebenen Produkte Lewatit®, Amberlite®, Dowex®, Duolite®, Nafion®, Permutit®, Chempro® oder Imac®.

Im erfindungsgemäßen Verfahren werden die sauren Ionenaustauscher vorzugsweise in getrockneter Form eingesetzt. Die Trocknung kann durch Wärme und/oder Vakuum erreicht werden. Weiterhin kann eine Trocknung durch Waschen mit hydrophilen Flüssigkeiten wie beispielsweise des im Verfahren eingesetzten Carbonsäureanhydrids erfolgen oder durch azeotrope Destillation mit organischen Lösungsmitteln wie Toluol, Xylol oder Methylenchlorid.

Die Katalysatoren können z.B. als Pulver oder Formkörper eingesetzt werden und nach der Umsetzung z.B. durch Filtration, Sedimentation oder Zentrifugieren abgetrennt werden.

In einer bevorzugten Ausführungsform der Erfindung wird der Katalysator als Festbettkatalysator eingesetzt.

Für den Fall der Anordnung als Festbett werden die homogenen Katalysatoren vorzugsweise auf einem Träger aufgebracht und als Formkörper, z.B. als Kugeln, Zylinder, Stäbchen, Hohlzylinder, Ringe usw. eingesetzt.

Die heterogenen Katalysatoren werden gegebenenfalls durch Wärme, gegebenenfalls im Vakuum, gegebenenfalls durch Waschen mit hydrophilen organischen Flüssigkeiten wie beispielsweise das eingesetzte Carbonsäureanhydrid oder gegebenenfalls durch azeotrope Destillation mit organischen Flüssigkeiten wie Toluol, Xylol oder Methylenchlorid getrocknet.

Bei Arbeiten in Lösung oder Suspension werden die Katalysatoren in Rührgefäßen in Mengen von 0,1 bis 100 Gew.-%, bevorzugt von 0,5 bis 90 Gew.-% und besonders bevorzugt von 1,0 bis 80 Gew.-%, bezogen auf Dibenzylether, verwendet.

Im Falle einer kontinuierlichen Fahrweise im Gegen- oder Gleichstrom oder in der Rieselphase am Festbettkatalysator werden Katalysatorbelastungen von 0,05 g bis 5000 g Dibenzylether pro Liter Katalysator pro Stunde, bevorzugt von 0,1 bis 4000 g /1 h und besonders bevorzugt von 1,0 bis 3000 g / 1 h verwendet.

In einer bevorzugten Ausführungsform der Erfindung wird eine oder mehrere, bevorzugt eine, Säure in einer Menge von 0,5 bis 100 Gew.-%, bezogen auf die Menge des Dibenzylethers, bei gelöstem oder suspendiertem Katalysator bzw. mit Belastungen von 1,0 bis 3000 g Dibenzylether pro Liter Katalysator pro Stunde bei der Anordnung als Festbettkatalysator eingesetzt.

Vorzugsweise wird das erfindungsgemäße Verfahren unter intensiver Durchmischung der Reaktionspartner durchgeführt. Eine intensive Durchmischung kann auf verschiedene, dem Fachmann bekannte Arten, etwa durch Rühren, Düsen, Strombrecher, statische Mischer, Pumpen, turbulente Strömungen in engen Röhren oder durch Ultraschall erreicht werden.

Derartige Vorrichtungen sind in Ullmann's Encyclopedia of Industrial Chemistry, 5. Auflage, Band B, Unit Operations, Abschnitte 25, 26, B4 S. 569 bis 570, Verlag Chemie, Weinheim 1988, näher beschrieben.

Eine bevorzugte Ausführungsform des erfindungsgemäßen Verfahrens ist indem man in eine Mischung oder Suspension des heterogenen Katalysators und des Carbonsäureanhydrids Dibenzylether zugibt und nach Beendigung der Reaktion den Katalysator durch z.B. Filtration oder Zentrifugieren abtrennt.

Eine weitere bevorzugte Durchführungsform ist das Gleichstromverfahren, bei der Dibenzylether und Carbonsäureanhydrid im Gleichstrom beispielsweise von oben her auf eine in einem Rohr angeordnete Katalysatorschüttung aufgebracht und unten am Fuß des Rohres Carbonsäurebenzylester abgezogen werden.

In einer weiteren bevorzugten Ausführungsform des erfindungsgemäßen Verfahrens wird dieses in der Rieselphase durchgeführt und der Katalysator liegt als Festbettkatalysator vor. Bevorzugt befindet sich die Katalysatorschüttung in einem senkrecht stehenden Rohrreaktor, welcher vorzugsweise Zwischenböden zur besseren Verteilung des Flüssigkeitsstroms und zur besseren Benetzung der Katalysatorschüttung enthält.

Die Aufarbeitung kann sowohl im Falle des suspendierten Katalysators als auch bei der Festbettverfahrensvariante so durchgeführt werden, dass ein mit Wasser nicht mischbares Lösungsmittel, vorzugsweise Toluol, zu den Reaktionsprodukten gegeben wird. Nach der Abtrennung der organischen Phase, welche den rohen Carbonsäurebenzylester enthält, kann diese beispielsweise durch Destillation weiter gereinigt werden.

Das erfindungsgemäße Verfahren kann diskontinuierlich, kontinuierlich oder teilkontinuierlich durchgeführt werden.

Die Temperatur, bei der das erfindungsgemäße Verfahren durchgeführt wird, beträgt vorzugsweise 10 bis 200, besonders bevorzugt 25 bis 190, ganz besonders bevorzugt 30 bis 180°C.

Der Druck, bei dem das erfindungsgemäße Verfahren durchgeführt wird, beträgt vorzugsweise 0,1 bis 50 bar.

Bei der Durchführung der Reaktion oberhalb 140°C muss entsprechend dem Dampfdruck unter erhöhtem Druck gearbeitet werden. Der benötigte Überdruck ist dann wenigstens gleich dem Dampfdruck des Reaktionsgemisches. Es kann bis etwa 50 bar betragen, vorzugsweise bis 25 bar.

Das Molverhältnis Dibenzylether zu Carbonsäureanhydrid im erfindungsgemäßen Verfahren beträgt vorzugsweise 1 : 1 bis 1 : 50.

Gegebenenfalls kann das erfindungsgemäße Verfahren unter einem üblichen Schutzgas wie beispielsweise Stickstoff, Helium oder Argon, durchgeführt werden.

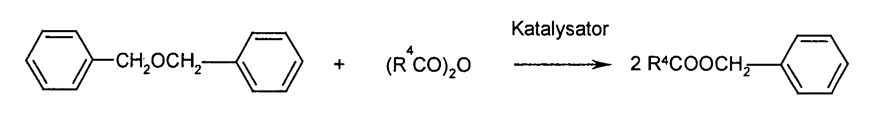

Nach dem erfindungsgemäßen Verfahren erhält man Carbonsäurebenzylester in guten Ausbeuten bei hohem Umsatz und guter Selektivität. Das erfindungsgemäße Verfahren ist ohne hohen apparativen Aufwand einfach durchführbar.

### Beispiele

Die Prozentangaben der nachfolgenden Beispiele beziehen sich auf das Gewicht.

### Beispiel 1

99,2 g (0,5 Mol) Dibenzylether, 51,0 g (0,5 Mol) Essigsäureanhydrid und 1,0 g konz. H₂SO₄ wurden in einem Kolben mit Strombrecher und Flügelrührer unter kräftigem Rühren (250 U/min) und unter Stickstoff bei 80°C erhitzt. Nach 7 Stunden Reaktionszeit wurde rasch abgekühlt, die organische Phase nach Zugabe von Toluol und Wasser abgetrennt und gaschromatographisch analysiert. Das Reaktionsgemisch enthielt Benzylacetat und Dibenzylether im Verhältnis 43 zu 43.

### Beispiel 2

Beispiel 1 wurde wiederholt, aber mit einer Reaktionstemperatur von 100°C. Die Reaktionszeit betrug 7 Stunden. Das Reaktionsgemisch enthielt Benzylacetat und Dibenzylether im Verhältnis 72 zu 10.

### Beispiel 3

Beispiel 1 wurde wiederholt, aber mit einer Reaktionstemperatur von 120°C. Die Reaktionszeit betrug 5 Stunden. Das Reaktionsgemisch enthielt Benzylacetat und Dibenzylether im Verhältnis 81 zu 3.

### Beispiel 4

Beispiel 1 wurde wiederholt, aber mit 0,5 g konz. H₂SO₄ und einer Reaktionszeit von 7 Stunden. Das Reaktionsgemisch enthielt Benzylacetat und Dibenzylether im Verhältnis 47 zu 37.

### Beispiel 5

Beispiel 1 wurde wiederholt, aber mit 1,0 g Lewatit SC 102 und einer Reaktionstemperatur von 60°C. Die Reaktionszeit betrug 5 Stunden. Das Reaktionsgemisch enthielt Benzylacetat und Dibenzylether im Verhältnis 31 zu 51.

### Beispiel 6

Beispiel 1 wurde wiederholt, aber mit 3,0 g Lewatit SC 102 und einer Reaktionstemperatur von 80°C. Die Reaktionszeit betrug 1 Stunde. Das Reaktionsgemisch enthielt Benzylacetat und Dibenzylether im Verhältnis 75 zu 4.

### Beispiel 7

Beispiel 1 wurde wiederholt, aber mit 89,0 g (0,5 Mol) Chloressigsäureanhydrid und einer Reaktionstemperatur von 100°C. Die Reaktionszeit betrug 30 Minuten. Es wurde Chloressigsäurebenzylester mit einer Selektivität von 69 %, bezogen auf den Umsatz des Dibenzylethers, gebildet.

### Beispiel 8

Zu einer Mischung aus 53,6 g (0,525 Mol) Essigsäureanhydrid und 1,0 g konz. H₂SO₄ in einem Kolben mit Strombrecher und Flügelrührer tropft man bei 120°C unter kräftigem Rühren (250 U/min) und unter Stickstoff innerhalb 50 min 99,2 g (0,5 Mol) Dibenzylether zu. Nach 5 Stunden Reaktionszeit wurde rasch abgekühlt, die organische Phase nach Zugabe von Toluol und Wasser abgetrennt und gaschromatographisch analysiert. Das Reaktionsgemisch enthielt Benzylacetat und Dibenzylether im Verhältnis 74 zu 14.

### Beispiel 9

Beispiel 8 wurde wiederholt, aber mit einer Reaktionstemperatur von 140°C. Die Reaktionszeit betrug 2 Stunden. Das Reaktionsgemisch enthielt Benzylacetat und Dibenzylether im Verhältnis 82 zu 8.

### Beispiel 10

Beispiel 8 wurde wiederholt, aber mit 0,25 g Trifluormethansulfonsäure und einer Reaktionstemperatur von 100°C. Die Reaktionszeit betrug 1 Stunde. Das Reaktionsgemisch enthielt Benzylacetat und Dibenzylether im Verhältnis 84 zu 2.

### Beispiel 11

Beispiel 8 wurde wiederholt, aber mit 0,5 g Bortrifluorid-diethyletherat und einer Reaktionstemperatur von 100°C. Die Reaktionszeit betrug 2 Stunden. Das Reaktionsgemisch enthielt Benzylacetat und Dibenzylether im Verhältnis 73 zu 6.

### Beispiel 12

Beispiel 8 wurde wiederholt, aber mit 0,5 g Wolframatophosphorsäure und einer Reaktionstemperatur von 80°C. Die Reaktionszeit betrug 1 Stunde. Das Reaktionsgemisch enthielt Benzylacetat und Dibenzylether im Verhältnis 71 zu 6.

### Beispiel 13

Beispiel 8 wurde wiederholt, aber mit 2,0 g eines sulfatierten Kieselgels (75,0 g SO₃ / 1 SiO₂) und einer Reaktionstemperatur von 100°C. Die Reaktionszeit betrug 2 Stunden. Das Reaktionsgemisch enthielt Benzylacetat und Dibenzylether im Verhältnis 32 zu 60.

### Beispiel 14

Beispiel 8 wurde wiederholt, aber mit 2,0 g eines sulfatierten Kieselgels (20,0 g SO₃/1 SiO₂) und einer Reaktionstemperatur von 100°C. Die Reaktionszeit betrug 5 Stunden. Das Reaktionsgemisch enthielt Benzylacetat und Dibenzylether im Verhältnis 29 zu 60.

### Beispiel 15

Beispiel 8 wurde wiederholt, aber mit 2,0 g eines sulfatierten Tantaloxids (20,0 g SO₃ / 1 Ta₂O₅) und einer Reaktionstemperatur von 100 °C. Die Reaktionszeit betrug 6 Stunden. Das Reaktionsgemisch enthielt Benzylacetat und Dibenzylether im Verhältnis 35 zu 52.

### Beispiel 16

Beispiel 8 wurde wiederholt, aber mit 2,0 g eines sulfatierten Calciumsulfats (17,4 g SO₃ / 1 CaSO₄) und einer Reaktionstemperatur von 100 °C. Die Reaktionszeit betrug 6 Stunden. Das Reaktionsgemisch enthielt Benzylacetat und Dibenzylether im Verhältnis 43 zu 46.

### Beispiel 17

Beispiel 8 wurde wiederholt, aber mit 2,0 g eines Katalysators, hergestellt durch Aufbringen von 10 g Trifluormethansulfonsäure auf 1000 ml Kieselgel. Die Reaktionszeit betrug 5 Stunden. Das Reaktionsgemisch enthielt Benzylacetat und Dibenzylether im Verhältnis 28 zu 60.

### Beispiel 18

Beispiel 8 wurde wiederholt, aber mit 2,0 g eines Katalysators, hergestellt durch Aufbringen von 75,0 g Wolframatophosphorsäure auf 1000 ml TiO₂ und einer Reaktionstemperatur von 100°C. Die Reaktionszeit betrug 2 Stunden. Das Reaktionsgemisch enthielt Benzylacetat und Dibenzylether im Verhältnis 55 zu 23.

### Beispiel 19

Beispiel 8 wurde wiederholt, aber mit 68,3 g (0,525 mol) Propionsäureanhydrid. Die Reaktionszeit betrug 6 Stunden. Das Reaktionsgemisch enthielt Benzylpropionat und Dibenzylether im Verhältnis 53 zu 34.

### Beispiel 20

Beispiel 8 wurde wiederholt, aber mit 118,8 g (0,525 mol) Benzoesäureanhydrid. Die Reaktionszeit betrug 3 Stunden. Das Reaktionsgemisch enthielt Benzylbenzoat und Dibenzylether im Verhältnis 78 zu 4.

### Beispiel 21 (Aufarbeitung)

Beispiel 9 wurde wiederholt und nach 3 Stunden Laufzeit aufgearbeitet. Nach Neutralisation und destillativer Auftrennung des Reaktionsgemisches isoliert man bei 107 - 109 °C / 33 mbar 91 g (61 %) Benzylacetat mit einer Reinheit von 99,1 %. Der Vorlauf enthält noch 14 g (9 %) Benzylacetat, der Rückstand 13 g (9 %) Benzylacetat und 5 g (5 %) Dibenzylether. Ausbeute 79 % (83 % bezogen auf umgesetzten Dibenzylether).

## Patentansprüche

1. Verfahren zur Herstellung von Carbonsäurebenzylestem der Formel 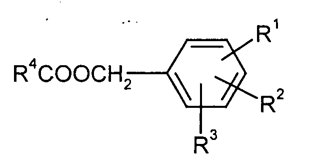 worin
R¹ bis R³ gleich oder verschieden sind und für C₁-C₆-Alkyl, C₁-C₆-Alkoxy-, C₁-C₆-Halogenalkyl, C₁-C₆-Halogenalkoxy-, CN, CO(C₁-C₆-Alkyl), NO₂ oder Halogen und
R⁴ für Wasserstoff, C₁-C₂₀-Alkyl, C₂-C₂₀-Alkenyl, C₇-C₁₄-Aralkyl, C₆-C₁₂-Aryl, C₁-C₆-Halogenalkyl, C₂-C₆-Halogenalkenyl- oder C₆-C₁₂-Halogenaryl,
stehen,
aus Dibenzylethern, **dadurch gekennzeichnet, dass** Dibenzylether der Formel 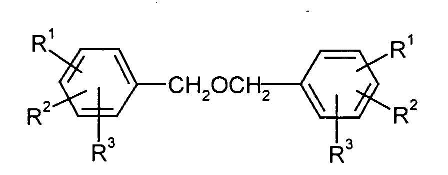 worin
R¹, R² und R³ die obengenannte Bedeutung haben,
oder Gemische aus Dibenzylethern und Benzylalkoholen der Formel 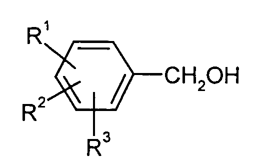 worin
R¹, R² und R³ die obengenannte Bedeutung haben
mit Carbonsäureanhydriden der Formel
(R⁴CO)₂O
worin
R⁴ die obengenannte Bedeutung hat,
in Gegenwart mindestens einer Säure als Katalysator, der gegebenenfalls auf einem Träger aufgebracht sein kann, umgesetzt werden.

2. Verfahren nach Anspruch 1, **dadurch gekennzeichnet, dass** die Säuren anorganische Säuren, organische Säuren oder Lewissäuren mit einem pH-Wert von 1 bis 6 sind.

3. Verfahren nach Anspruch 1 oder 2, **dadurch gekennzeichnet, dass** die Säuren Schwefeltrioxid, Schwefelsäure, Chlorwasserstoff, Bromwasserstoff, Iodwasserstoff, Flusssäure, Perchlorsäure, Chlorsulfonsäure, Phosphorsäure, Trifluoressigsäure, Methansulfonsäure, Ethansulfonsäure, Benzolsulfonsäure, 4-Toluolsulfonsäure, Bortrifluorid, Aluminiumchlorid, Aluminiumbromid, Aluminiumiodid, Zinkchlorid, Zinnchlorid, Titanchlorid oder Zirkonchlorid, gegebenenfalls aufgebracht auf einem oder mehreren Trägern, sind.

4. Verfahren nach Anspruch 1, **dadurch gekennzeichnet, dass** Heteropolysäuren der Formel (I) eingesetzt werden
AₐX_{b}M_{c}O_{d} (I)
worin
A für Protonen und/oder für Metallkationen steht
X für P, Si, B, Ge, As, I, Se oder Te steht
M für W, Mo, V oder Cr steht
a für 3, 4, 5 oder 6 steht, so dass die Elektroneutralität der Heteropolysäuren oder deren Salze gegeben ist
b für 1 oder 2 steht
c für 12 oder 18 steht und
d für 40 oder 62 steht,
gegebenenfalls aufgebracht auf einem oder mehreren Trägern.

5. Verfahren nach Anspruch 4, **dadurch gekennzeichnet, dass** A für ein Kation aus der Reihe Wasserstoff, Lithium, Natrium, Kalium, Rubidium, Caesium, Mangan, Nickel, Cobalt, Kupfer oder Lanthan steht.

6. Verfahren nach Anspruch 4, **dadurch gekennzeichnet, dass** als Heteropolysäuren Molybdatophosphorsäure, Wolframatophosphorsäure, Vanadatophosphorsäure, Molybdatokieselsäure, Wolframatokieselsäure oder Vanadatokieselsäure, gegebenenfalls aufgebracht auf einem oder mehreren Trägern, eingesetzt werden.

7. Verfahren nach Anspruch 1, **dadurch gekennzeichnet, dass** als Säure ein saurer Ionenaustauscher eingesetzt wird.

8. Verfahren nach Anspruch 7, **dadurch gekennzeichnet, dass** als Säure ein Sulfonsäure-Gruppen tragendes Polymer eingesetzt wird.

9. Verfahren nach Anspruch 1, **dadurch gekennzeichnet, dass** die Umsetzung bei einer Temperatur von 10 bis 200°C durchgeführt wird.

10. Verfahren nach Anspruch 1, **dadurch gekennzeichnet, dass** als Säure ein sulfatiertes Oxid eingesetzt wird.
